# EUROPÄISCHE PATENTANMELDUNG

(11) **EP 2 409 986 A1**
(43) Veröffentlichungstag der Anmeldung: **25.01.2012**
(21) Anmeldenummer: 10170690.1
(22) Anmeldetag: 23.07.2010
(51) Int. Cl.: C07K 14/395, C12N 15/81

(54) **Erfindung betreffend Flokkulation von Hefezellen**

(71) Anmelder: PHILIPPS-UNIVERSITÄT MARBURG, 35037 Marburg (DE)
(72) Erfinder: Brückner, Stefan, 35034 Marburg (DE); Essen, Lars-Oliver, 35043 Marburg (DE); Moesch, Hans-Ulrich, 35037 Marburg (DE); Veelders, Maik, 35037 Marburg (DE)
(74) Vertreter: Stumpf, Peter

(57) **Zusammenfassung**

Die vorliegende Erfindung beschreibt Mutationen in der Flo5A-Domän Flokkulinen industrieller Hefen, die zu einer gezielten Modifikation der Substratspezifität und von Flokkulinproteinen führen. Insbesondere Flo5A-Q98A Mutante zeigt überraschenderweise *in vivo* einen *NewFlo*-Typ der Flokkulationsinhibition, durch eine Inhibition auch durch Glucose, ohne jedoch insgesamt an Flokkulationsfähigkeit zu verlieren.

Weiterhin wird eine Verfahren zur Detektion Flo5-tragender Hefezellen in einer Zellsuspension bereitgestellt.

SEQUENCE LISTING

<110>  Philipps-universität Marburg

<120>  Erfindung betreffend Flokkulation

<130>  TM 476

<160>  36

<170>  PatentIn version 3.4

<210>  1
<211>  249
<212>  PRT
<213>  Saccharomyces cerevisiae

<220>
<221>  Flo5A-Domäne
<222>  (1)..(249)

<400>  1

Ser Gly Ala Thr Glu Ala Cys Leu Pro Ala Gly Gln Arg Lys Ser Gly
1               5                   10                  15

Met Asn Ile Asn Phe Tyr Gln Tyr Ser Leu Lys Asp Ser Ser Thr Tyr
            20                  25                  30

Ser Asn Ala Ala Tyr Met Ala Tyr Gly Tyr Ala Ser Lys Thr Lys Leu
        35                  40                  45

Gly Ser Val Gly Gly Gln Thr Asp Ile Ser Ile Asp Tyr Asn Ile Pro
    50                  55                  60

Cys Val Ser Ser Ser Gly Thr Phe Pro Cys Pro Gln Glu Asp Ser Tyr
65                  70                  75                  80

Gly Asn Trp Gly Cys Lys Gly Met Gly Ala Cys Ser Asn Ser Gln Gly
                85                  90                  95

Ile Ala Tyr Trp Ser Thr Asp Leu Phe Gly Phe Tyr Thr Thr Pro Thr
            100                 105                 110

Asn Val Thr Leu Glu Met Thr Gly Tyr Phe Leu Pro Pro Gln Thr Gly
        115                 120                 125

Ser Tyr Thr Phe Ser Phe Ala Thr Val Asp Asp Ser Ala Ile Leu Ser
    130                 135                 140

Val Gly Gly Ser Ile Ala Phe Glu Cys Cys Ala Gln Glu Gln Pro Pro
145                 150                 155                 160

Ile Thr Ser Thr Asn Phe Thr Ile Asn Gly Ile Lys Pro Trp Asp Gly
                165                 170                 175

Ser Leu Pro Asp Asn Ile Thr Gly Thr Val Tyr Met Tyr Ala Gly Tyr
            180                 185                 190

Tyr Tyr Pro Leu Lys Val Val Tyr Ser Asn Ala Val Ser Trp Gly Thr
        195                 200                 205

Leu Pro Ile Ser Val Glu Leu Pro Asp Gly Thr Thr Val Ser Asp Asn
    210                 215                 220

Phe Glu Gly Tyr Val Tyr Ser Phe Asp Asp Asp Leu Ser Gln Ser Asn
225                 230                 235                 240

Cys Thr Ile Pro Asp Pro Ser Ile His
                245

<210>  2
<211>  249
<212>  PRT
<213>  Saccharomyces cerevisiae

<220>
<221>  Q98AFlo5A-Mutante
<222>  (1)..(249)

<400>  2

Ser Gly Ala Thr Glu Ala Cys Leu Pro Ala Gly Gln Arg Lys Ser Gly
1               5                   10                  15

Met Asn Ile Asn Phe Tyr Gln Tyr Ser Leu Lys Asp Ser Ser Thr Tyr
            20                  25                  30

Ser Asn Ala Ala Tyr Met Ala Tyr Gly Tyr Ala Ser Lys Thr Lys Leu
        35                  40                  45

Gly Ser Val Gly Gly Gln Thr Asp Ile Ser Ile Asp Tyr Asn Ile Pro
    50                  55                  60

Cys Val Ser Ser Ser Gly Thr Phe Pro Cys Pro Ala Glu Asp Ser Tyr
65                  70                  75                  80

Gly Asn Trp Gly Cys Lys Gly Met Gly Ala Cys Ser Asn Ser Gln Gly
                85                  90                  95

Ile Ala Tyr Trp Ser Thr Asp Leu Phe Gly Phe Tyr Thr Thr Pro Thr
            100                 105                 110

Asn Val Thr Leu Glu Met Thr Gly Tyr Phe Leu Pro Pro Gln Thr Gly
        115                 120                 125

Ser Tyr Thr Phe Ser Phe Ala Thr Val Asp Asp Ser Ala Ile Leu Ser
    130                 135                 140

Val Gly Gly Ser Ile Ala Phe Glu Cys Cys Ala Gln Glu Gln Pro Pro
145                 150                 155                 160

Ile Thr Ser Thr Asn Phe Thr Ile Asn Gly Ile Lys Pro Trp Asp Gly
                165                 170                 175

Ser Leu Pro Asp Asn Ile Thr Gly Thr Val Tyr Met Tyr Ala Gly Tyr
            180                 185                 190

Tyr Tyr Pro Leu Lys Val Val Tyr Ser Asn Ala Val Ser Trp Gly Thr
        195                 200                 205

Leu Pro Ile Ser Val Glu Leu Pro Asp Gly Thr Thr Val Ser Asp Asn
    210                 215                 220

Phe Glu Gly Tyr Val Tyr Ser Phe Asp Asp Asp Leu Ser Gln Ser Asn
225                 230                 235                 240

Cys Thr Ile Pro Asp Pro Ser Ile His
                245

<210>  3
<211>  249
<212>  PRT
<213>  Saccharomyces cerevisiae

<220>
<221>  D160/D161 Mutante Flo5A
<222>  (1)..(249)

<400>  3

Ser Gly Ala Thr Glu Ala Cys Leu Pro Ala Gly Gln Arg Lys Ser Gly
1               5                   10                  15

Met Asn Ile Asn Phe Tyr Gln Tyr Ser Leu Lys Asp Ser Ser Thr Tyr
            20                  25                  30

Ser Asn Ala Ala Tyr Met Ala Tyr Gly Tyr Ala Ser Lys Thr Lys Leu
        35                  40                  45

Gly Ser Val Gly Gly Gln Thr Asp Ile Ser Ile Asp Tyr Asn Ile Pro
    50                  55                  60

Cys Val Ser Ser Ser Gly Thr Phe Pro Cys Pro Gln Glu Asp Ser Tyr
65                  70                  75                  80

Gly Asn Trp Gly Cys Lys Gly Met Gly Ala Cys Ser Asn Ser Gln Gly
                85                  90                  95

Ile Ala Tyr Trp Ser Thr Asp Leu Phe Gly Phe Tyr Thr Thr Pro Thr
            100                 105                 110

Asn Val Thr Leu Glu Met Thr Gly Tyr Phe Leu Pro Pro Gln Thr Gly
        115                 120                 125

Ser Tyr Thr Phe Ser Phe Ala Thr Val Ala Ala Ser Ala Ile Leu Ser
    130                 135                 140

Val Gly Gly Ser Ile Ala Phe Glu Cys Cys Ala Gln Glu Gln Pro Pro
145                 150                 155                 160

Ile Thr Ser Thr Asn Phe Thr Ile Asn Gly Ile Lys Pro Trp Asp Gly
                165                 170                 175

Ser Leu Pro Asp Asn Ile Thr Gly Thr Val Tyr Met Tyr Ala Gly Tyr
            180                 185                 190

Tyr Tyr Pro Leu Lys Val Val Tyr Ser Asn Ala Val Ser Trp Gly Thr
        195                 200                 205

Leu Pro Ile Ser Val Glu Leu Pro Asp Gly Thr Thr Val Ser Asp Asn
    210                 215                 220

Phe Glu Gly Tyr Val Tyr Ser Phe Asp Asp Asp Leu Ser Gln Ser Asn
225                 230                 235                 240

Cys Thr Ile Pro Asp Pro Ser Ile His
                245

<210>  4
<211>  248
<212>  PRT
<213>  Saccharomyces cerevisiae

<220>
<221>  D202T Mutante Flo5A
<222>  (1)..(248)

<400>  4

Ser Gly Ala Thr Glu Ala Cys Leu Pro Ala Gly Gln Arg Lys Ser Gly
1               5                   10                  15

Met Asn Ile Asn Phe Tyr Gln Tyr Ser Leu Lys Asp Ser Ser Thr Tyr
            20                  25                  30

Ser Asn Ala Ala Tyr Met Ala Tyr Gly Tyr Ala Ser Lys Thr Lys Leu
        35                  40                  45

Gly Ser Val Gly Gly Gln Thr Asp Ile Ser Ile Asp Tyr Asn Ile Pro
    50                  55                  60

Cys Val Ser Ser Ser Gly Thr Phe Pro Cys Pro Gln Glu Asp Ser Tyr
65                  70                  75                  80

Gly Asn Trp Gly Cys Lys Gly Met Gly Ala Cys Ser Asn Ser Gln Gly
                85                  90                  95

Ile Ala Tyr Trp Ser Thr Asp Leu Phe Gly Phe Tyr Thr Thr Pro Thr
            100                 105                 110

Asn Val Thr Leu Glu Met Thr Gly Tyr Phe Leu Pro Pro Gln Thr Gly
        115                 120                 125

Ser Tyr Thr Phe Ser Phe Ala Thr Val Asp Asp Ser Ala Ile Leu Ser
    130                 135                 140

Val Gly Gly Ser Ile Ala Phe Glu Cys Cys Ala Gln Glu Gln Pro Pro
145                 150                 155                 160

Ile Thr Ser Thr Asn Phe Thr Ile Asn Gly Ile Lys Pro Trp Asp Gly
                165                 170                 175

Ser Leu Pro Thr Asn Thr Gly Thr Val Tyr Met Tyr Ala Gly Tyr Tyr
            180                 185                 190

Tyr Pro Leu Lys Val Val Tyr Ser Asn Ala Val Ser Trp Gly Thr Leu
        195                 200                 205

Pro Ile Ser Val Glu Leu Pro Asp Gly Thr Thr Val Ser Asp Asn Phe
    210                 215                 220

Glu Gly Tyr Val Tyr Ser Phe Asp Asp Asp Leu Ser Gln Ser Asn Cys
225                 230                 235                 240

Thr Ile Pro Asp Pro Ser Ile His
                245

<210>  5
<211>  249
<212>  PRT
<213>  Saccharomyces cerevisiae

<220>
<221>  Mutante Y222F Flo5A
<222>  (1)..(249)

<400>  5

Ser Gly Ala Thr Glu Ala Cys Leu Pro Ala Gly Gln Arg Lys Ser Gly
1               5                   10                  15

Met Asn Ile Asn Phe Tyr Gln Tyr Ser Leu Lys Asp Ser Ser Thr Tyr
            20                  25                  30

Ser Asn Ala Ala Tyr Met Ala Tyr Gly Tyr Ala Ser Lys Thr Lys Leu
        35                  40                  45

Gly Ser Val Gly Gly Gln Thr Asp Ile Ser Ile Asp Tyr Asn Ile Pro
    50                  55                  60

Cys Val Ser Ser Ser Gly Thr Phe Pro Cys Pro Gln Glu Asp Ser Tyr
65                  70                  75                  80

Gly Asn Trp Gly Cys Lys Gly Met Gly Ala Cys Ser Asn Ser Gln Gly
                85                  90                  95

Ile Ala Tyr Trp Ser Thr Asp Leu Phe Gly Phe Tyr Thr Thr Pro Thr
            100                 105                 110

Asn Val Thr Leu Glu Met Thr Gly Tyr Phe Leu Pro Pro Gln Thr Gly
        115                 120                 125

Ser Tyr Thr Phe Ser Phe Ala Thr Val Asp Asp Ser Ala Ile Leu Ser
    130                 135                 140

Val Gly Gly Ser Ile Ala Phe Glu Cys Cys Ala Gln Glu Gln Pro Pro
145                 150                 155                 160

Ile Thr Ser Thr Asn Phe Thr Ile Asn Gly Ile Lys Pro Trp Asp Gly
                165                 170                 175

Ser Leu Pro Asp Asn Ile Thr Gly Thr Val Tyr Met Tyr Ala Gly Tyr
            180                 185                 190

Tyr Tyr Pro Leu Lys Val Val Phe Ser Asn Ala Val Ser Trp Gly Thr
        195                 200                 205

Leu Pro Ile Ser Val Glu Leu Pro Asp Gly Thr Thr Val Ser Asp Asn
    210                 215                 220

Phe Glu Gly Tyr Val Tyr Ser Phe Asp Asp Asp Leu Ser Gln Ser Asn
225                 230                 235                 240

Cys Thr Ile Pro Asp Pro Ser Ile His
                245

<210>  6
<211>  249
<212>  PRT
<213>  Saccharomyces cerevisiae

<220>
<221>  Mutante N224A Flo5A
<222>  (1)..(249)

<400>  6

Ser Gly Ala Thr Glu Ala Cys Leu Pro Ala Gly Gln Arg Lys Ser Gly
1               5                   10                  15

Met Asn Ile Asn Phe Tyr Gln Tyr Ser Leu Lys Asp Ser Ser Thr Tyr
            20                  25                  30

Ser Asn Ala Ala Tyr Met Ala Tyr Gly Tyr Ala Ser Lys Thr Lys Leu
        35                  40                  45

Gly Ser Val Gly Gly Gln Thr Asp Ile Ser Ile Asp Tyr Asn Ile Pro
    50                  55                  60

Cys Val Ser Ser Ser Gly Thr Phe Pro Cys Pro Gln Glu Asp Ser Tyr
65                  70                  75                  80

Gly Asn Trp Gly Cys Lys Gly Met Gly Ala Cys Ser Asn Ser Gln Gly
                85                  90                  95

Ile Ala Tyr Trp Ser Thr Asp Leu Phe Gly Phe Tyr Thr Thr Pro Thr
            100                 105                 110

Asn Val Thr Leu Glu Met Thr Gly Tyr Phe Leu Pro Pro Gln Thr Gly
        115                 120                 125

Ser Tyr Thr Phe Ser Phe Ala Thr Val Asp Asp Ser Ala Ile Leu Ser
    130                 135                 140

Val Gly Gly Ser Ile Ala Phe Glu Cys Cys Ala Gln Glu Gln Pro Pro
145                 150                 155                 160

Ile Thr Ser Thr Asn Phe Thr Ile Asn Gly Ile Lys Pro Trp Asp Gly
                165                 170                 175

Ser Leu Pro Asp Asn Ile Thr Gly Thr Val Tyr Met Tyr Ala Gly Tyr
            180                 185                 190

Tyr Tyr Pro Leu Lys Val Val Tyr Ser Ala Ala Val Ser Trp Gly Thr
        195                 200                 205

Leu Pro Ile Ser Val Glu Leu Pro Asp Gly Thr Thr Val Ser Asp Asn
    210                 215                 220

Phe Glu Gly Tyr Val Tyr Ser Phe Asp Asp Asp Leu Ser Gln Ser Asn
225                 230                 235                 240

Cys Thr Ile Pro Asp Pro Ser Ile His
                245

<210>  7
<211>  249
<212>  PRT
<213>  Saccharomyces cerevisiae

<220>
<221>  Mutante S227A Flo5A

<222>  (1)..(249)

<400>  7

Ser Gly Ala Thr Glu Ala Cys Leu Pro Ala Gly Gln Arg Lys Ser Gly
1               5                   10                  15

Met Asn Ile Asn Phe Tyr Gln Tyr Ser Leu Lys Asp Ser Ser Thr Tyr
            20                  25                  30

Ser Asn Ala Ala Tyr Met Ala Tyr Gly Tyr Ala Ser Lys Thr Lys Leu
        35                  40                  45

Gly Ser Val Gly Gly Gln Thr Asp Ile Ser Ile Asp Tyr Asn Ile Pro
    50                  55                  60

Cys Val Ser Ser Ser Gly Thr Phe Pro Cys Pro Gln Glu Asp Ser Tyr
65                  70                  75                  80

Gly Asn Trp Gly Cys Lys Gly Met Gly Ala Cys Ser Asn Ser Gln Gly
                85                  90                  95

Ile Ala Tyr Trp Ser Thr Asp Leu Phe Gly Phe Tyr Thr Thr Pro Thr
            100                 105                 110

Asn Val Thr Leu Glu Met Thr Gly Tyr Phe Leu Pro Pro Gln Thr Gly
        115                 120                 125

Ser Tyr Thr Phe Ser Phe Ala Thr Val Asp Asp Ser Ala Ile Leu Ser
    130                 135                 140

Val Gly Gly Ser Ile Ala Phe Glu Cys Cys Ala Gln Glu Gln Pro Pro
145                 150                 155                 160

Ile Thr Ser Thr Asn Phe Thr Ile Asn Gly Ile Lys Pro Trp Asp Gly
                165                 170                 175

Ser Leu Pro Asp Asn Ile Thr Gly Thr Val Tyr Met Tyr Ala Gly Tyr
            180                 185                 190

Tyr Tyr Pro Leu Lys Val Val Tyr Ser Asn Ala Val Ala Trp Gly Thr
        195                 200                 205

Leu Pro Ile Ser Val Glu Leu Pro Asp Gly Thr Thr Val Ser Asp Asn
    210                 215                 220

Phe Glu Gly Tyr Val Tyr Ser Phe Asp Asp Asp Leu Ser Gln Ser Asn
225                 230                 235                 240

Cys Thr Ile Pro Asp Pro Ser Ile His
                245

<210>  8
<211>  249
<212>  PRT
<213>  Saccharomyces cerevisiae

<220>
<221>  W228L Mutante Flo5A
<222>  (1)..(249)

<400>  8

Ser Gly Ala Thr Glu Ala Cys Leu Pro Ala Gly Gln Arg Lys Ser Gly
1               5                   10                  15

Met Asn Ile Asn Phe Tyr Gln Tyr Ser Leu Lys Asp Ser Ser Thr Tyr
            20                  25                  30

Ser Asn Ala Ala Tyr Met Ala Tyr Gly Tyr Ala Ser Lys Thr Lys Leu
        35                  40                  45

Gly Ser Val Gly Gly Gln Thr Asp Ile Ser Ile Asp Tyr Asn Ile Pro
    50                  55                  60

Cys Val Ser Ser Ser Gly Thr Phe Pro Cys Pro Gln Glu Asp Ser Tyr
65                  70                  75                  80

Gly Asn Trp Gly Cys Lys Gly Met Gly Ala Cys Ser Asn Ser Gln Gly
                85                  90                  95

Ile Ala Tyr Trp Ser Thr Asp Leu Phe Gly Phe Tyr Thr Thr Pro Thr
            100                 105                 110

Asn Val Thr Leu Glu Met Thr Gly Tyr Phe Leu Pro Pro Gln Thr Gly
        115                 120                 125

Ser Tyr Thr Phe Ser Phe Ala Thr Val Asp Asp Ser Ala Ile Leu Ser
    130                 135                 140

Val Gly Gly Ser Ile Ala Phe Glu Cys Cys Ala Gln Glu Gln Pro Pro
145                 150                 155                 160

Ile Thr Ser Thr Asn Phe Thr Ile Asn Gly Ile Lys Pro Trp Asp Gly
                165                 170                 175

Ser Leu Pro Asp Asn Ile Thr Gly Thr Val Tyr Met Tyr Ala Gly Tyr
            180                 185                 190

Tyr Tyr Pro Leu Lys Val Val Tyr Ser Asn Ala Val Ser Leu Gly Thr
        195                 200                 205

Leu Pro Ile Ser Val Glu Leu Pro Asp Gly Thr Thr Val Ser Asp Asn
    210                 215                 220

Phe Glu Gly Tyr Val Tyr Ser Phe Asp Asp Asp Leu Ser Gln Ser Asn
225                 230                 235                 240

Cys Thr Ile Pro Asp Pro Ser Ile His
                245

<210>  9
<211>  29
<212>  DNA
<213>  Artificial

<220>
<223>  primer for PCR

<220>
<221>  Primer FLO11-1
<222>  (1)..(29)

<400>  9
aaagagctca gttggaaaac ccaaagccg                                        29

<210>  10
<211>  34
<212>  DNA
<213>  Artificial

<220>
<223>  Primer für PCR

<220>
<221>  Primer FLO11-4
<222>  (1)..(34)

<400>  10
aaagagctct aatgatacaa ttccaacatg ttcg                                  34

<210>  11
<211>  41
<212>  DNA
<213>  Artificial

<220>
<223>  Primer für PCR

<220>
<221>  Primer FLO11-5
<222>  (1)..(41)

<400>  11
aaagagctca tagattgtga caacaattgt gctccagtac c                          41

<210>  12
<211>  34
<212>  DNA
<213>  Artificial

<220>
<223>  Primer für PCR

<220>
<221>  Primer FLO11-6
<222>  (1)..(34)

<400>  12
aaagagctct gcagttggaa aacccaaagc tgag                                34


<210>  13
<211>  29
<212>  DNA
<213>  Artificial

<220>
<223>  Primer für PCR


<220>
<221>  Primer FLO5-1
<222>  (1)..(29)

<400>  13
aaagagctca cagaggcgtg cttaccagc                                      29


<210>  14
<211>  36
<212>  DNA
<213>  Artificial

<220>
<223>  Primer für PCR


<220>
<221>  Primer FLO5-2
<222>  (1)..(36)

<400>  14
aaagagctca ctactagcac tatcacaact accacc                              36


<210>  15
<211>  32
<212>  DNA
<213>  Artificial

<220>
<223>  Primer für PCR


<220>
<221>  Primer FLO5-3
<222>  (1)..(32)

<400>  15
aaagagctca tgtattgaag gatcagggat ag                                  32


<210>  16
<211>  31
<212>  DNA
<213>  Artificial

<220>
<223>  Primer für PCR

<220>
<221>  Primer FLO5-4
<222>  (1)..(31)

<400>  16
aaagagctca ataattgcca gcaataagga c                                    31


<210>  17
<211>  32
<212>  DNA
<213>  Artificial

<220>
<223>  Primer für PCR


<220>
<221>  Primer FLO5-5
<222>  (1)..(32)

<400>  17
aaaggatccg ccagttcttt agtgattaca gg                                   32


<210>  18
<211>  23
<212>  DNA
<213>  Artificial

<220>
<223>  Primer für PCR


<220>
<221>  Primer FLO5-6
<222>  (1)..(23)

<400>  18
attgaattcg aagtgttgtt tgc                                             23


<210>  19
<211>  25
<212>  DNA
<213>  Artificial

<220>
<223>  Primer für PCR


<220>
<221>  FLO5-AA23
<222>  (1)..(25)

<400>  19
ccatatgtca ggagccacag aggcg                                           25


<210>  20
<211>  36
<212>  DNA
<213>  Artificial

<220>
<223>  Primer für PCR

<220>
<221>  Primer FLO5-AA271
<222>  (1)..(36)

<400>  20
actcgagtta atgtattgaa ggatcaggga tagtac                                36


<210>  21
<211>  30
<212>  DNA
<213>  Artificial

<220>
<223>  Primer für PCR


<220>
<221>  Primer FLO5D160A-D161A-1
<222>  (1)..(30)

<400>  21
tttgcaacag tagctgcttc tgcaatttta                                       30


<210>  22
<211>  30
<212>  DNA
<213>  Artificial

<220>
<223>  Primer für PCR


<220>
<221>  Primer FLO5D160A-D161A-2
<222>  (1)..(30)

<400>  22
taaaattgca gaagcagcta ctgttgcaaa                                       30


<210>  23
<211>  25
<212>  DNA
<213>  Artificial

<220>
<223>  primer für PCR


<220>
<221>  Primer FLO5D202T-1
<222>  (1)..(25)

<400>  23
gaagtctccc taccaatatc acagg                                            25


<210>  24
<211>  25
<212>  DNA
<213>  Artificial

<220>
<223>  Primer für PCR

<220>
<221>  Primer FLO5D202T-2
<222>  (1)..(25)

<400>  24
cctgtgatat tggtagggag acttc                                          25


<210>  25
<211>  23
<212>  DNA
<213>  Artificial

<220>
<223>  Primer für PCR


<220>
<221>  Primer FLO5K194A-1
<222>  (1)..(23)

<400>  25
caatggtatc gcgccatggg atg                                            23


<210>  26
<211>  23
<212>  DNA
<213>  Artificial

<220>
<223>  Primer für PCR


<220>
<221>  Primer FLO5K194A-2
<222>  (1)..(23)

<400>  26
catcccatgg cgcgatacca ttg                                            23


<210>  27
<211>  24
<212>  DNA
<213>  Artificial

<220>
<223>  Primer für PCR


<220>
<221>  Primer FLO5N224A-1
<222>  (1)..(24)

<400>  27
gttgtttact ccgctgccgt ttcc                                           24


<210>  28
<211>  24
<212>  DNA
<213>  Artificial

<220>
<223>  Primer für PCR

<220>
<221>  FLO5N224A-2
<222>  (1)..(24)

<400>  28
ggaaacggca gcggagtaaa caac                                              24


<210>  29
<211>  25
<212>  DNA
<213>  Artificial

<220>
<223>  Primer für PCR


<220>
<221>  Primer FLO5Q98A-1
<222>  (1)..(25)

<400>  29
ttccttgtcc tgcagaagat tccta                                             25


<210>  30
<211>  25
<212>  DNA
<213>  Artificial

<220>
<223>  Primer für PCR


<220>
<221>  Primer FLO5Q98A-2
<222>  (1)..(25)

<400>  30
taggaatctt ctgcaggaca aggaa                                             25


<210>  31
<211>  23
<212>  DNA
<213>  Artificial

<220>
<223>  Primer für PCR


<220>
<221>  Primer FLO5S227A-1
<222>  (1)..(23)

<400>  31
caatgccgtt gcctggggca cgc                                               23


<210>  32
<211>  23
<212>  DNA
<213>  Artificial

<220>
<223>  Primer für PCR

<220>
<221>  Primer FLO5S227A-2
<222>  (1)..(23)

<400>  32
gcgtgcccca ggcaacggca ttg                                              23


<210>  33
<211>  22
<212>  DNA
<213>  Artificial

<220>
<223>  Primer für PCR


<220>
<221>  Primer FLO5W228L-1
<222>  (1)..(22)

<400>  33
gccgtttcct tgggcacgct tc                                               22


<210>  34
<211>  22
<212>  DNA
<213>  Artificial

<220>
<223>  Primer für PCR


<220>
<221>  Primer FLO5W228L-2
<222>  (1)..(22)

<400>  34
gaagcgtgcc caaggaaacg gc                                               22


<210>  35
<211>  25
<212>  DNA
<213>  Artificial

<220>
<223>  Primer für PCR


<220>
<221>  Primer FLO5Y222F-1
<222>  (1)..(25)

<400>  35
ctgaaggttg ttttctccaa tgccg                                            25


<210>  36
<211>  25
<212>  DNA
<213>  Artificial

<220>
<223>  Primer für PCR

<220>
<221>  Primer FLO5Y222F-2
<222>  (1)..(25)

<400>  36
cggcattgga gaaaacaacc ttcag                                          25

## Beschreibung

Die vorliegende Erfindung betrifft die Flokkulation von industriellen Hefen, insbesondere die zelladhäsionsvermittelnde Flo5A-Domäne von industriellen Hefen und die Herstellung von industriellen Hefen mit spezifischen Mutationen und veränderten Flokkulationseigenschaften, sowie die Verwendung von Flo5A-spezifischen Antikörpern in einem Verfahren zur Detektion Flo5A-tragender industrieller Hefen in einer Zellsuspension.

### Beschreibung und Einleitung des allgemeinen Gebietes der Erfindung

Industrielle Hefen sind Hefen, die ein Anwendungsspektrum in industriellen Prozessen aufweisen.dazu gehört z.B. die Bäckerhefe und obergärige Bierhefe Saccharomyces cerevisiae, die untergärige Bierhefen Saccharomyces carlsbergensis und Saccharomyces uvarum, die Spalthefe Schizosaccharomyces pombe, Candida utilis, die zur Herstellung von Kefir verwendet wird, Pichia pastoris, die in biotechnischen Verfahren zur Produktion von Proteinen benutzt wird. Diese Hefen sind ideale Systeme für die Produktion von Fremdproteinen. und die in diesen industriellen Hefen hergestellten Proteine sind identisch oder nahezu identisch mit den Proteinen von Tieren oder Menschen.

Proteine, wie Flokkuline vermitteln in industriellen Hefen z.B. Saccharomyces cerevisiae die asexuelle Aggregation von ansonsten einzelligen Hefen unter nahrungslimitierten oder anderen ungünstigen Bedingungen. Diese Flokkulation wird seit Jahrhunderten im Bereich des Bierbrauens verwendet, um nach Abschluss der Gärung eines untergärigen Bieres (z.B. Pilsener Art) die Hefe kostengünstig und umweltfreundlich durch einfache Filtration vom Jungbier zu trennen. Weiterhin findet Flokkulation mit S. *cerevisiae* Verwendung in der industriellen Ethanolproduktion und neuerdings auch in der Produktion von Hormonen und Metaboliten wie Insulin und L-Milchsäure. Auch bei diesen Anwendungen ist eine einfache Abtrennung der Hefe nach Abschluss der Fermentation erwünscht. Trotz der immensen wirtschaftlichen Bedeutung sind die molekularen Zusammenhänge, die zur Flokkulation führen, bisher weitgehend unbekannt und deshalb war es nicht möglich, gezielt spezifische Mutationen an Schlüsselpositionen des Proteins zur Verbesserung der Flokkulation vorzunehmen oder zielgerichtete Hefemutanten mit veränderten Flokkulinen und veränderten Flokkulationseigenschaften bereitzustellen.

Im Stand der Technik ist bisher bekannt, dass für die Flokkulation von Hefen wie *S. carlsbergensis* eine LgFlo1 Domäne verantwortlich ist. Zwar wurde LgFlo1 aus *S. carlsbergensis* erfolgreich kristallisiert, allerdings erfolgte keine darauf aufbauende Struktur- und Funktionsanalyse, so dass der molekulare Mechanismus der Flokkulation der LgFlo1 Domäne und der Flokkulation überhaupt bislang noch ungeklärt ist.

In der industriellen Anwendung ermöglicht die Flokkulation eine einfache Abtrennung der Hefe vom fermentierten Produkt. Aus bisher ungeklärter Ursache verlieren Brauhefen nach mehreren neuen Brauansätzen, bei denen immer mit Hefe aus früheren Ansätzen überimpft wird, die Fähigkeit Flokkuline zu produzieren und damit zu flokkulieren. Dies stellt ein entscheidendes Problem dar, denn die Flokkulationsfähigkeit von Hefen, die im industriellen Prozess eingesetzt werden sollen, muss ständig überprüft werden. Bisher erfolgt die Bestimmung der Flokkulationsfähigkeit von Hefen z.B. mit Hilfe des Helm- oder Gillilandtests, der nur von geschultem Personal mit großer Erfahrung durchgeführt werden kann. Helm- oder Gillilandtest sind halb quantitative Versuche, bei denen die Flokkulationsfähigkeit einer Hefekultur optisch beurteilt wird. Die Durchführung optischer Tests in Bezug auf die Flokkulationsfähigkeit führt jedoch zu ungenauen oder sogar falschen Ergebnissen, die zu einem finanziellen Verlust durch Ausfall oder zumindest Verzögerung einer Fermentation führen können.

Alternativ werden die Durchflusszytometrie, Proteomanalyse sowie die mRNA-Profilierung eingesetzt, diese Methoden erfordern allerdings allesamt einen erheblichen experimentellen Aufwand, sind vergleichsweise teuer und nicht in industriellem Maßstab durchzuführen. Eine weitere Methode die Flokkulationsfähigkeit einer Hefekultur zu testen, beruht auf dem genetischen Nachweis der Flokkulationsgene mit einer quantitativen Polymerase-Ketten-Reaktion, in der ein Teil der LgFlo1-DNA-Sequenz als Primer verwendet wird, der eine Amplifizierung des Genes ermöglicht, wenn eines vorhanden ist. Mit diesem Verfahren werden auch andere Flokkulationsgene z.B. die Flo5-DNA-Sequenz oder Flo1- DNA-Sequenz nachgewiesen. Diese Verfahren sind jedoch ziemlich teuer, erfordern eine umfangreiche Laborausstattung und sind nur von geschultem Personal und unter besonders sterilen Bedingungen durchführbar, um keine falsch-positiven Amplifikate durch Verunreinigungen zu erhalten.

Neben der Bestimmung der Flokkulationsfähigkeit ist ein weiterer wichtiger Parameter bei der Flokkulation die Beeinflussung des Flokkulationszeitpunktes.

Der Flokkulationszeitpunkt z.B. von Brauhefe ist von essentieller Bedeutung für einen erfolgreichen Prozess, z.B. den Gärungsprozess. Findet die Flokkulation verfrüht statt, so entsteht eine hängende Gärung, die nicht mehr bis zum gewünschten Endpunkt durchlaufen werden kann. Findet die Flokkulation zu spät oder nur unvollständig statt, so ist die einfache Separation der Hefe vom Produkt nicht mehr gegeben.

Eine Beeinflussung des Flokkulationszeitpunktes erfolgt bisher meistens unter empirischen Gesichtspunkten und eine gezielte Beeinflussung des Flokkulationszeitpunktes ist derzeit in der Praxis nicht möglich. Die Möglichkeit, das für das Flokkulin Flo1 codierende Gen unter den Promotor der ADH zu stellen,wie in US 5,585,271 beschrieben, führt ausschließlich zu einer hochfrequenten Expression des Flokkulin Flo1 und damit einer starken Flokkulation der Hefezellen.

Es ist bekannt, dass sich die Flokkulation mit Flokkulinen Flo1 und Flo5, der sogenannte FIo5/Flo1-Typ Flokkulation durch Zugabe von Mannose inhibieren lässt, wohingegen die LgFlo1- oder *NewFlo*-Typ Flokkulation zusätzlich zu Mannose auch durch Glukose, Maltose und Saccharose inhibierbar ist.

Eine gezielte Regulation der Flokkulation und damit eine gezielte Beeinflussung des Flokkulationszeitpunktes ist derzeit jedoch nicht möglich.

### Aufgabe

Aufgabe der vorliegenden Erfindung ist es die Nachteile im Stand der Technik zu beseitigen und eine bessere Regulation der Flokkulation von Hefen zu ermöglichen.

### Lösung der Aufgabe

Diese Aufgabe wird erfindungsgemäß durch die Ansprüche gelöst, insbesondere durch die Bereitstellung von Hefen, die Flo5A-Mutationen tragen, sowie ein Verfahren, mit dem Flo5-tragende Hefezellen detektiert werden.

Eigene wissenschaftliche Untersuchungen der Erfinder zeigen, dass die in Laborstämmen vorhandenen Flokkuline Flo1, Flo5, Flo9 und Flo10 eine große Sequenzhomologie in der N-terminalen A-Domäne (=Flo5A-Domäne) aufweisen.

Die Erfinder zeigen erstmals, dass überraschenderweise bereits die N-terminale A-Domäne dieser Flokkuline ausreichend für die Flokkulationsfähigkeit der Hefen ist.

Es ist den Wissenschaftlern gelungen, die atomare Struktur und Funktionsweise dieser Flo5A-Domäne aufzuklären und zu zeigen, dass ein neuartiges Faltungsmuster eines Lektins unter direkter Einbeziehung eines Calciumions in die Zuckerbindung, vorliegt.

Erfindungsgemäß werden verschiedene Mutationen in der Flo5A-Domäne bereitgestellt und in Hefezellen transformiert, die eine verbesserte Regulation der Flokkulation ermöglichen.

Erfindungsgemäß wird ein neuer nativer Ligand der Flo5A-Domäne bereitgestellt.

Erfindungsgemäß wird ein Verfahren zur Detektion von Flo5-tragenden Zellen insbesondere Hefezellen bereitgestellt. In diesem Verfahren wird ein polyklonaler Antikörper eingesetzt, der eine spezifische Bindungsfähigkeit an die Flo5A-Domäne aufweist.

Erfindungsgemäß werden verbesserte analytische Verfahren zur Beeinflussung des Flokkulationszeitpunktes und zur Bestimmung der Flokkulationsfähigkeit bereitgestellt. Dazu wird der polyklonale Antikörper mit einer spezifischen Bindungsfähigkeit an die Flo5A-Domäne, in einem ELISA eingesetzt, um die Intensität mit der eine Hefekultur Flokkuline produziert auf schnelle und kostengünstige Art festzustellen.

Mit dem erfindungsgemäßen Verfahren ist erstmals eine routinemäßige Beeinflussung und Steuerung des Flokkulationszeitpunktes möglich. Durch gezielte Mutagenese wird die Spezifität der Flokkuline sowie die Stärke der von ihnen vermittelten Kohlenhydratbindung verändert.

### Ausführungsbeispiele

### 1. Struktur der Flo5A-Domäne (Wildtyp)

Die wissenschaftlichen Arbeiten zeigen eine hochaufgelöste atomare Struktur der für die Flokkulation essentiellen und ausreichenden A-Domäne des Flokkulins Flo5 (=Flo5A-Domäne). Die Flo5A-Domäne ist für die Flokkulation essentiellen und ausreichend.

### 2. Mutationen der Flo5A-Domäne

Ausgehend von der hoch aufgelösten atomare Struktur der für die Flokkulation essentiellen und ausreichenden A-Domäne des Flokkulins Flo5 wurden verschiedene Mutanten der A-Domäne des Flokkulins Flo5 mit unterschiedlichen Bindungseigenschaften für verschiedene Kohlenhydrate hergestellt, charakterisiert und hinsichtlich ihrer Flokkulationseigenschaften analysiert.

Erfindungsgemäße Mutationen im Flo5A-Flokkulin:

| **Flo5A-Flokkulin** | **Art der Mutation** |
|---|---|
| Flo5A-Q98A | Substitution |
| | Q (Glutamin) an Position 98 gegen A (Alanin) |
| Flo5A-D202T | Substitution |
| | D (Asparaginsäure) an Position 202 gegen T (Threonin) |
| Flo5A-N224A | Substitution |
| | N (Asparagin) an Position 224 gegen A (Alanin) |
| Flo5A-D160A/D161A | Substitution |
| | D (Asparaginsäure) an Position 160 und 161 gegen A (Alanin) |
| FloSA-Y222F | Substitution |
| | Y (Tyrosin) an Position 222 gegen F (Phenylalanin) |
| Flo5A-S227A | Substitution |
| | S (Serin) an Position 227gegen A (Alanin) |
| Flo5A-W228L | Substitution |
| | W (Tryptophan) an Position 228 gegen L (Leucin) |

### 3. Plasmid Konstruktion zur Herstellung der Flo5-Mutanten

Hefestämme die als Ausgangsmaterial verwendet werden:

| **Hefestamm** | **Genotyp** | **Quelle** |
|---|---|---|
| RH2662 | *MAT***a** *flo11*Δ*::kanR ura3-52* | Braus et al., 2003 |
| RH2754 | *MAT***a** *ura3-52 leu2:: hisG his3::hisG trp1::hisG* | Köhler et al., 2002 |

Plasmide die verwendet werden:

| **Plasmid** | **Genotyp** |
|---|---|
| BHUM1301 | *P_{FLO11}-FLO11¹⁻²⁴-T_{FLO11}* in YCplac33 |
| BHUM1323 | *P_{FLO5}-FLO5-T_{FLO5}* in pCR-BLUNT II-TOPO |
| BHUM1327 | *P_{FLO11}-FLO11¹⁻²⁴-FLO11 ²¹⁴⁻¹³⁶⁰ -T_{FLO11}*in YCplac33 |
| BHUM1435 | *P_{FLO11}-FLO11¹⁻²⁴ -FLO5²⁶⁻¹⁰⁷⁵-T_{FLO11}* in YCplac33 |
| BHUM1437 | *P_{FLO11}-FLO11 ¹⁻²⁵-FLO5 ²⁶⁻²⁷¹- FLO11 ²¹⁴⁻¹³⁶⁰-T_{FLO11}* in YCplac33 |
| BHUM1505 | *P_{FLO11}-FLO11¹⁻²⁴*-*FLO5^{272- 1075}- T_{FLO11}* in YCplac33 |
| BHUM1514 | *FLO5²³⁻²⁷¹* in pET-28a(+) |
| BHUM1526 | FLO5^{*23-271*,*D202T*} in pET-28a(+) |
| BHUM1528 | *FLO5^{23-271,Y222F}* in pET-28a(+) |
| BHUM1530 | *FLO5^{23-271,N224A}* in pET-28a(+) |
| BHUM1532 | *FLO5^{23-271,Q98A}* in pET-28a(+) |
| BHUM1533 | *FLO5^{23-271, D161A}* in pET-28a(+) |
| BHUM1535 | *FLO5^{23-271,K194A}* in pET-28a(+) |
| BHUM1537 | *FLO5^{23-271, W228L}* in pET-28a(+) |
| BHUM1540 | *FLO5^{23-271,S227A}* in pET-28a(+) |
| BHUM1567 | *P_{FLO11}*-*FLO11¹⁻²⁵-FLO5^{26-271,D160A-D161A}-FLO11²¹⁴⁻¹³⁶⁰-T_{FLO11}* in YCplac33 |
| BHUM1568 | *P_{FLO11}-FLO11¹⁻²⁵-FLO5^{23-271,D202T}-FLO11²¹⁴⁻¹³⁶⁰ -T_{FLO11}*in YCplac33 |
| BHUM1570 | *P_{FLO11}-FLO11¹⁻²⁵-FLO5²⁶⁻²⁷¹, ^{Y222F}-FLO11²¹⁴⁻¹³⁶⁰ - T_{FLO11}* in YCplac33 |
| BHUM1571 | *FLO11-FLO11¹⁻²⁵-FLO5^{26-271,S227A}-FLO5^{26-271,S227A}-T_{FLO11}* in YCplac33 |
| BHUM1573 | *P_{FLO11}-FLO11¹⁻²⁵FLO5^{26-271,W228L}-FLO11²¹⁴⁻¹³⁶⁰-T_{FLO11}*in YCplac33 |
| BHUM1581 | *P_{FLO11}-FLO11¹⁻²⁵FLO5^{26-271,N224A}-FLO11²¹⁴⁻¹³⁶⁰-T_{FLO11}*in YCplac33 |
| BHUM1583 | *P_{FLO11}-FLO11¹⁻²⁵FLO5^{26-271,Q98A}-FLO11²¹⁴⁻¹³⁶⁰-T_{FLO11}* in YCplac33 *P_{FLO11}-FLO11¹⁻²⁵FLO5^{26-271,q98a}-FLO11²¹⁴⁻¹³⁶⁰-T*_{*FLO11* in YCplac33} |
| BHUM1585 | *P_{FLO11}-FLO11¹⁻²⁵FLO5^{26-271,k194a}-FLO11²¹⁴⁻¹³⁶⁰-T_{FLO11}*in YCplac33 |
| pET-28a(+) | *P_{T7} 6HIS lacl Kan* |
| pME2519 | *P_{FLO5}-FLO11- T_{FLO11}* in YCplac33 |

DNA-Primer die verwendet werden:

| **Primer** | **Sequenz 5' zu 3'** |
|---|---|
| Seq. ID No. 9 FLO11-1 | AAAGAGCTCAGTTGGAAAACCCAAAGCCG |
| Seq. ID No. 10 FLO11-4 | AAAGAGCTCTAATGATACAATTCCAACATGTTCG |
| Seq. ID No. 11 FLO11-5 | AAAGAGCTCATAGATTGTGACAACAATTGTGCTCCAGTACC |
| Seq. ID No. 12 FLO11-6 | AAAGAGCTCTGCAGTTGGAAAACCCAAAGCTGAG |
| Seq. ID No. 13 FL05-1 | AAAGAGCTCACAGAGGCGTGCTTACCAGC |
| Seq. ID No. 14 FL05-2 | AAAGAGCTCACTACTAGCACTATCACAACTACCACC |
| Seq. ID No. 15 FL05-3 | AAAGAGCTCATGTATTGAAGGATCAGGGATAG |
| Seq. ID No. 16 FL05-4 | AAAGAGCTCAATAATTGCCAGCAATAAGGAC |
| Seq. ID No. 17 FL05-5 | AAAGGATCCGCCAGTTCTTTAGTGATTACAGG |
| Seq. ID No. 18 FL05-6 | ATTGAATTCGAAGTGTTGTTTGC |
| Seq. ID No. 19 FL05-AA23 | CCATATGTCAGGAGCCACAGAGGCG |
| Seq. ID No. 20 FL05-AA271 | ACTCGAGTTAATGTATTGAAGGATCAGGGATAGTAC |
| Seq. ID No. 21 FLO5^{D160A- D161A-}1 | TTTGCAACAGTAGCTGCTTCTGCAATTTTA |
| Seq. ID No. 22 FLO5^{D160A- D161A}-2 | TAAAATTGCAGAAGCAGCTACTGTTGCAAA |
| Seq. ID No. 23 FLO5^{D202T}-1 | GAAGTCTCCCTACCAATATCACAGG |
| Seq. ID No. 24 FLO5^{D202T}-2 | CCTGTGATATTGGTAGGGAGACTTC |
| Seq. ID No. 25 FLO5^{K194A}-1 | CAATGGTATCGCGCCATGGGATG |
| Seq. ID No. 26 FLO5^{K194A}-2 | CATCCCATGGCGCGATACCATTG |
| Seq. ID No. 27 FLO5^{N224A}-1 | GTTGTTTACTCCGCTGCCGTTTCC |
| Seq. ID No. 28 FLO5^{N224A}-2 | GGAAACGGCAGCGGAGTAAACAAC |
| Seq. ID No. 29 FLO5^{Q98A}-1 | TTCCTTGTCCTGCAGAAGATTCCTA |
| Seq. ID No. 30 FLO5^{Q98A} -2 | TAGGAATCTTCTGCAGGACAAGGAA |
| Seq. ID No. 31 FLO5^{S227A}-1 | CAATGCCGTTGCCTGGGGCACGC |
| Seq. ID No. 32 FLO5^{S227A}-2 | GCGTGCCCCAGGCAACGGCATTG |
| Seq. ID No. 33 FLO5^{W228L}-1 | GCCGTTTCCTTGGGCACGCTTC |
| Seq. ID No. 34 FLO5 ^{W228L}-2 | GAAGCGTGCCCAAGGAAACGGC |
| Seq. ID No. 35 FLO5^{Y222F}-1 | CTGAAGGTTGTTTTCTCCAATGCCG |
| Seq. ID No. 36 FLO5^{Y222F}-2 | CGGCATTGGAGAAAACAACCTTCAG |

BHUM1323 wird hergestellt durch PCR Amplifikation des gesamten FL05 Genes von chromosomaler Hefe DNA (Stamm RH2754), wobei Primer FL05-5 und FL05-6 verwendet werden. Das resultierende DNA Fragment wird nach einem dem Fachmann bekannt Verfahren in den Vector pCR-BLUNT II-TOPO (Invitrogen, Karlsruhe) kloniert.

BHUM1514 wird hergestellt durch PCR Amplifikation des FL05 Fragments das für die Aminosäuren 23 to 271 kodiert, wobei Plasmid BHUM1323 als Vorlage dient und das Primerpaar FLO5-AA23/FLO5-AA271 verwendet wird. Das resultierende DNA Fragment wird nach einem dem Fachmann bekannt Verfahren in den E. coli Expressionsvektor pET-28a(+) (Merck, Darmstadt, Germany) kloniert unter Nutzung von Restriktionsenzymen z.B. Ndel und Xhol.

BHUM1301 trägt den FLO11 Promoter, die FLO11 Sekretionssignalsequenz (Aminosäure 1 bis 24) und den FLO11 Terminator wird hergestellt durch PCR des ganzen Vektor (whole Vector PCR) mit Plasmid pME2519 als Vorlage und Primer FLO11-1 und FLO11-4 nach Restriktionsspaltung mit einem Restriktionsenzymen z.B. Sacl und anschließender Ligation.

BHUM1327 trägt eine FLO11 Variant mit fehlender A Domäne (Aminosäuren 25 bis 213) und wird hergestellt durch PCR des ganzen Vektor (whole Vector PCR) mit Plasmid pME2519 als Vorlage und den Primer pair FL011-5/FL011-6.

BHUM1435 trägt das *FLO5* Gen (Aminosäuren 26 bis 1075) fusioniert mit der Signalsequenz des *FLO11* Gen und angetrieben vom *FLO11* Promoter. das entsprechende *FLO5* Genfragment wird über PCR amplifiziert ausgehend von chromosomaler DNA (RH2754) und Verwendung der Primer FL05-1 und FL05-4. Anschließend erfolgt die insertion des resultierende *FLO5*Genfragment in die Sacl Schnittstelle des Plasmid BHUM1301.

BHUM1505 wird hergestellt durch Amplification des *FLO5* ORF, dem die A Domäne fehlt (Aminosäuren 272 bis 1075). Dazu wird das Primerpaar FL05-2/FL05-4 und chromosomale DNA des Hefestamm RH2754 verwendet. Das resultierende Genfragment wird in die Sacl Schnittstelle des Plasmid BHUM1301 eingebracht. Plasmid BHUM1437 trägt ein *FLO5A-FLO11BC* Fusionsgen unter der Kontrolle des *FLO11* Promoters, das durch Amplifikation des *FLO5A* Genfragment (kodierend Aminosäuren 26 to 271) von chromosomaler DNA (RH2754) entstand mittels Primer FL05-1 und FL05-3 und nachfolgender Insertion des resultierenden Fragmentes in die Sacl Schnittstelle des Plasmid BHUM1327.

Die Plasmide BHUM1526, BHUM1528, BHUM1530, BHUM1532, BHUM1533, BHUM1535, BHUM1537 und BHUM1540 entstanden durch PCR-basierte site directed mutagenesis ausgehend von Plasmid BHUM1514 mittel dem Fachmann bekanntem "QuickChange Site-Directed Mutagenesis Kit" (Stratagene, Amsterdam, Netherlands) und entsprechenden *FL0₅* Primerpaaren (FLO5^{D160A-D161A}-1 und FLO5^{D160A-D161A}-2_{,} FLO5^{D202T}-1 und FLO5^{D202T}-2 FLO5^{K194A}-1 und FLO5^{K194A}-2, FLO5^{N224A}-1 und FLO5N^{224A}-2, FLO5^{Q98A}-1 und FLO5^{Q98A}-2 FLO5^{S227A}-1 und FLO5^{S227A}-2, FLO5^{W228L}-1 und FLO5^{W228L}-2, FLO5^{Y222F}-1 und FLO5^{Y222F}-2), die die entsprechenden Aminosäuresubstitution einbringen.

Die Plasmide BHUM1567, BHUM1568, BHUM1570, BHUM1571, BHUM1573 und BHUM1581 werden ebenfalls durch die dem Fachmann bekannte site directed mutagenesis-Methode hergestellt, wobei die entsprechenden *FLO5* Primerpaare mit den angegebenen Aminosäuresubstitution und BHUM1437 als Vorlage eingesetzt werden.

Die Plasmide BHUM1583 und BHUM1585 entstanden durch Amplifikation des mutierten *FLO5A* Fragment mit Primern FL05-1 und FL05-3 von BHUM1532 oder BHUM1535 und anschließender Insertion in die Sacl Schnittstelle des Plasmid BHUM1327.

### 4. Eigenschaften des Flo5A Wildtyp und der Flo5A Mutanten:

### Flo5A Wildtyp:

Aminosäuresequenz des Flo5A Wildtypes.

Seq. ID No. 1 Wildtyp (23-271):

SGATEACLPAGQRKSGMNINFYQYSLKDSSTYSNAAYMAYGYASKTKLGSVGG
QTDISIDYNIPCVSSSGTFPCPQEDSYGNWGCKGMGACSNSQGIAYWSTDLFGF
YTTPTNVTLEMTGYFLPPQTGSYTFSFATVDDSAILSVGGSIAFECCAQEQPPITS
TNFTINGIKPWDGSLPDNITGTVYMYAGYYYPLKVVYSNAVSWGTLPISVELPDG
TTVSDNFEGYVYSFDDDLSQSNCTIPDPSIH

Die Flokkulation dieses Flokkulin dient als Referenz. Sie ist durch Mannose fast vollständig, durch Glukose jedoch nur schwach inhibierbar.

*In vitro* K_{D} (Mannose) = 29,3 ± 3,6 mM

*In vitro* K_{D} ( -1,2-Mannobiose) = 3,5 ± 0,3 mM

### Q98A:

Aminosäuresequenz des Flo5A Q98A Mutante, wobei die Mutation fett hervorgehoben ist.

Seq. ID No. 2 Q98A:

SGATEACLPAGQRKSGMNINFYQYSLKDSSTYSNAAYMAYGYASKTKLGSVGG
QTDISIDYNIPCVSSSGTFPCPAEDSYGNWGCKGMGACSNSQGIAYWSTDLFGF
YTTPTNVTLEMTGYFLPPQTGSYTFSFATVDDSAILSVGGSIAFECCAQEQPPITS

TNFTINGIKPWDGSLPDNITGTVYMYAGYYYPLKVVYSNAVSWGTLPISVELPDG
TTVSDNFEGYVYSFDDDLSQSNCTIPDPSIH

Diese Mutante weist eine zum Wildtyp von Flo5A vergleichbare Flokkulationsstärke auf, jedoch lässt sich die Flokkulation sowohl durch Mannose als auch durch Glucose in ähnlichem Maße inhibieren.

### D160A/D161 A:

Aminosäuresequenz des Flo5A D160A/D161 A Mutante, wobei die Mutation fett hervorgehoben ist.

Seq. ID No. 3 D160A/D161A:

SGATEACLPAGQRKSGMNINFYQYSLKDSSTYSNAAYMAYGYASKTKLGSVGG
QTDISIDYNIPCVSSSGTFPCPQEDSYGNWGCKGMGACSNSQGIAYWSTDLFGF
YTTPTNVTLEMTGYFLPPQTGSYTFSFATVAASAILSVGGSIAFECCAQEQPPITS
TNFTINGIKPWDGSLPDNITGTVYMYAGYYYPLKVVYSNAVSWGTLPISVELPDG
TTVSDNFEGYVYSFDDDLSQSNCTIPDPSIH

Diese Mutante weist keine aktive Flokkulation auf, da das Calciumbindungsmotiv zerstört ist.

### D202T:

Aminosäuresequenz des Flo5A D202T Mutante, wobei die Mutation fett hervorgehoben ist.

Seq. ID No. 4 D202T:

SGATEACLPAGQRKSGMNINFYQYSLKDSSTYSNAAYMAYGYASKTKLGSVGG
QTDISIDYNIPCVSSSGTFPCPQEDSYGNWGCKGMGACSNSQGIAYWSTDLFGF
YTTPTNVTLEMTGYFLPPQTGSYTFSFATVDDSAILSVGGSIAFECCAQEQPPITS
TNFTINGIKPWDGSLPTNTGTVYMYAGYYYPLKVVYSNAVSWGTLPISVELPDGT
TVSDNFEGYVYSFDDDLSQSNCTIPDPSIH

Diese Mutante weist eine im Vergleich zum Wildtyp FloA reduzierte Flokkulationsstärke auf, die leicht verstärkt Mannose- und deutlich verstärkt Glukose-sensitiv reagiert.

*In vitro* K_{D} (Mannose) = 15,8 ± 2,6 mM

### Y222F:

Aminosäuresequenz des Flo5A Y222F, wobei die Mutation fett hervorgehoben ist. Seq. ID No. 5 Y222F:

SGATEACLPAGQRKSGMNINFYQYSLKDSSTYSNAAYMAYGYASKTKLGSVGG
QTDISIDYNIPCVSSSGTFPCPQEDSYGNWGCKGMGACSNSQGIAYWSTDLFGF
YTTPTNVTLEMTGYFLPPQTGSYTFSFATVDDSAILSVGGSIAFECCAQEQPPITS
TNFTINGIKPWDGSLPDNITGTVYMYAGYYYPLKVVFSNAVSWGTLPISVELPDG
TTVSDNFEGYVYSFDDDLSQSNCTIPDPSIH

Diese Mutante zeigt keine signifikanten Abweichungen vom Wildtyp.

### N224A:

Aminosäuresequenz des Flo5A N224A, wobei die Mutation fett hervorgehoben ist.

Seq. ID No. 6 N224A:

SGATEACLPAGQRKSGMNINFYQYSLKDSSTYSNAAYMAYGYASKTKLGSVGG
QTDISIDYNIPCVSSSGTFPCPQEDSYGNWGCKGMGACSNSQGIAYWSTDLFGF
YTTPTNVTLEMTGYFLPPQTGSYTFSFATVDDSAILSVGGSIAFECCAQEQPPITS
TNFTINGIKPWDGSLPDNITGTVYMYAGYYYPLKVVYSAAVSWGTLPISVELPDG
TTVSDNFEGYVYSFDDDLSQSNCTIPDPSIH

Diese Mutante zeigt eine stark eingeschränkte Flokkulation, die sowohl Mannose-und deutlich verstärkt Glukose-sensitiv reagiert.

### S227A:

Aminosäuresequenz des Flo5A S227A, wobei die Mutation fett hervorgehoben ist.

Seq. ID No. 7 S227A:

SGATEACLPAGQRKSGMNINFYQYSLKDSSTYSNAAYMAYGYASKTKLGSVGG
QTDISIDYNIPCVSSSGTFPCPQEDSYGNWGCKGMGACSNSQGIAYWSTDLFGF
YTTPTNVTLEMTGYFLPPQTGSYTFSFATVDDSAILSVGGSIAFECCAQEQPPITS
TNFTINGIKPWDGSLPDNITGTVYMYAGYYYPLKVVYSNAVAWGTLPISVELPDG
TTVSDNFEGYVYSFDDDLSQSNCTIPDPSIH

Diese Mutante zeigt eine dem Wildtyp vergleichbare Flokkulation, die leicht verstärkt Mannose- und leicht verstärkt Glukose-sensitiv reagiert.

*In vitro* K_{D} (Mannose) = 9,7 ± 0,6 mM

*In vitro K_{D}* ( -1,2-Mannobiose) = 1,6 ± 0,1 mM

### W228L:

Aminosäuresequenz des Flo5A W228L, wobei die Mutation fett hervorgehoben ist.

Seq. ID No. 8 W228L:

SGATEACLPAGQRKSGMNINFYQYSLKDSSTYSNAAYMAYGYASKTKLGSVGG
QTDISIDYNIPCVSSSGTFPCPQEDSYGNWGCKGMGACSNSQGIAYWSTDLFGF
YTTPTNVTLEMTGYFLPPQTGSYTFSFATVDDSAILSVGGSIAFECCAQEQPPITS
TNFTINGIKPWDGSLPDNITGTVYMYAGYYYPLKVVYSNAVSLGTLPISVELPDGT
TVSDNFEGYVYSFDDDLSQSNCTIPDPSIH

Diese Mutante zeigt eine ähnlich schwache Flokkulation wie die N224A-Mutante. Jedoch ist die Mannosesensitivität im Vergleich zum Wildtyp verringert und die Glukosesensitivität verstärkt.

### 5. Verfahren zur Detektion Flo5A-tragender Hefezellen

Zur schnellen und einfachen Detektion Flo5A-tragender Hefezellen in einer Zellsuspension wird ein Verfahren als immunologisches Nachweisverfahren in Form eines ELISA (enzyme-linked immunosorbent assay) bereitgestellt.

Die Detektion Flo5A-tragender Hefezellen erfolgt in einer Zellsuspension, die industrielle Hefen z.B. Saccharomyces cerevisiae enthält. Die Zellsuspension wird in die Vertiefungen einer kommerziell erhältlichen Mikrotiterplatte aufgebracht und die Hefezellen werden dort immobilisiert, z.B. durch eine ConcanavalinA-Beschichtung der Platte.

Alternativ findet in der Zellsuspension zuvor eine Bestimmung der optischen Dichte statt, um die Zellsuspension auf eine optische Dichte einzustellen, die geeignet ist, um den ELISA durchzuführen. Anschließend werden ungebunden Hefezellen und Bestandteile der Zellsuspension in einem Waschschritt entfernt und zurück bleiben nur die gebundenen Hefezellen. Im nächsten Schritt wird der für die Flo5A-Domäne spezifische Antikörper hinzugefügt. Dabei wird ein polyklonaler oder alternativ ein monoklonaler Antikörper verwendet. In einem Waschschritt wird ungebundener für die Flo5A-Domäne spezifischer Antikörper entfernt und zurück bleiben nur die gebundenen Hefezellen und daran gebundener für die Flo5A-Domäne spezifischer Antikörper. Im nächsten Schritt erfolgt die Detektion der Menge an Flo5A-tragenden Hefezellen über Zugabe eines markierten Zweitantikörper, der spezifisch Epitope des für die Flo5A-Domäne spezifischen Antikörpers erkennt und bindet. Hierzu trägt der Zweitantikörper eine Markierung z.B. in Form von colorimetrischer, radioaktiver oder vorzugsweise fluoreszierender Markierung.

Zur Kontrolle der Menge an immobilisierten Hefezellen wird im Anschluss eine Färbung der Hefezellen mittels Kristallviolett oder anderer zellanfärbender Agenzien durchgeführt. Eine zuvor nicht mit Zellsuspension behandelte Stelle der Mikrotiterplatte dient hierbei als Negativkontrolle.

Alternativ trägt der Zweitantikörper Ende ein Enzym z.B. Meerrettichperoxidase oder Glucoseoxidase und die Farbreaktion erfolgt durch Zugabe eines zum Enzym passenden chromogenen Substrates. Dieses Substrates wird vom Enzym zu einem Reaktionsprodukt umgesetzt, dessen Nachweis durch Farbumschlag, Fluoreszenz oder Chemiluminiszenz erfolgt.

Für quantitative Nachweise wird üblicherweise eine Serie mit bekannten Antigenkonzentrationen durchgeführt, um eine Kalibrierungskurve für das gemessene Signal (optische Extinktion, emittierte Intensität) zu erhalten.

Die Auswertung erfolgt, indem die Mikrotiterplatte in einem ELISA Reader ausgewertet wird. Vertiefungen, die eine positive Farbreaktion zeigen, weisen auf Flo5-tragende Hefezellen hin.

Das Verfahren wird auch zur Detektion von Flo5-tragende Hefezellen, die die Mutationen Flo5A-Q98A, Flo5A-D202T, Flo5A-N224A, Flo5A-D160A/D161 A, Flo5A-Y222F, FLoSA-S227A bzw. Flo5A-W228L aufweisen, eingesetzt.

Alternativ erfolgt der Nachweis von Flo5A Protein tragenden Zellen durch Immunfluoreszenz Mikroskopie

### 6. Herstellung des polyklonalen Anti-Flo5A Antikörpers

Der polyklonale Anti-Flo5A Antikörper wurde in Kaninchen hergestellt, durch Immunisierung mit 10 mg von gereinigtem Flo5 ²³⁻²⁷¹ Protein gemäß Standard Immunisierungsprotokoll (Pineda-Antikörper-Service, Berlin, Germany).

### 7. Rekombinante Überexpression der Flo5A Domäne

E. *coli* Origami2 (DE3) (Novagen), der BHUM1514 trägt, wird in LB-Medium mit IPTG (40 µM f.c.) für 3 Tage bei 12°C inkubiert. Nach Lyse wird der Überstand mit Protein über Ni-NTA Affinitätschromatographie (Qiagen) gereinigt und per Größenausschluss-Chromatographie mit superdex 200 prep grade material (GE Healthcare) in AM-Buffer (20 mM Tris/HCL, pH 8.0, 200 mM NaCl) isoliert.

### 8. Verfahren zum Einbringen von Flo5A-Domänen in Hefen

Das Verfahren basiert auf einem E. coli/Hefe-Shuttle Vektor, in dessen multiple cloning site folgende Elemente in dieser Reihenfolge eingefügt wurden: Gesamtpromotor von FL011, das Sekretionssignal von FL011, 3facher HA-Tag, die Restriktionsschnittstellen Sacll und Sacl, die BC Domäne von FL011, der Terminator von FL011.

Alternativ wird statt dem Gesamtpromotor von FLO11 der weniger komplex regulierten Promotor von PGK1 verwendet.

In die eingefügten Restriktionsstellen wird jeweils die flokkulations- oder adhäsions-vermittelnde Domäne (aus Hefe oder anderen Organismen), bzw. deren mutierte Teilbereiche eingefügt. insbesondere werden die Sequenzen mit Mutationen Flo5A-Q98A, Flo5A-D202T, Flo5A-N224A, Flo5A-D160A/D161A, Flo5A-Y222F, FLoSA-S227A bzw. Flo5A-W228L eingefügt.

Als Negativkontrolle kann das leere Ausgangskonstrukt fungieren, als Positivvergleich z.B. eine Fusion mit der FL05 A-Domäne (insbesondere dann, wenn die Flokkulation betrachtet werden soll) oder der FLO11 A-Domäne (wenn die Adhäsion beurteilen werden soll).

Durch den FLO11 Promotor und das Sekretionssignal ist sichergestellt, dass das Konstrukt in Hefe exprimiert und sekretiert wird.

Durch den 3fach HA-Tag benötigt man keinen speziellen Antikörper zum Nachweis des Fusionsproteins. Ein kommerziell erhältlicher Antikörper gegen HA (Hämagglutenin) genügt.

Durch die FLO11 BC-Domäne kann man mit der richtigen räumlichen Ausrichtung der flokkulations- bzw. adhäsions-vermittelnden A-Domäne rechnen.

Der alternative PGK1-Promotor macht die Expression gegebenenfalls unabhängig von der komplexen Regulation durch den FLO11 Promotor. Ein weiterer vollständig konstitutiver Promotor ist in Planung.

### Abbildungslegenden und Bezugszeichenliste

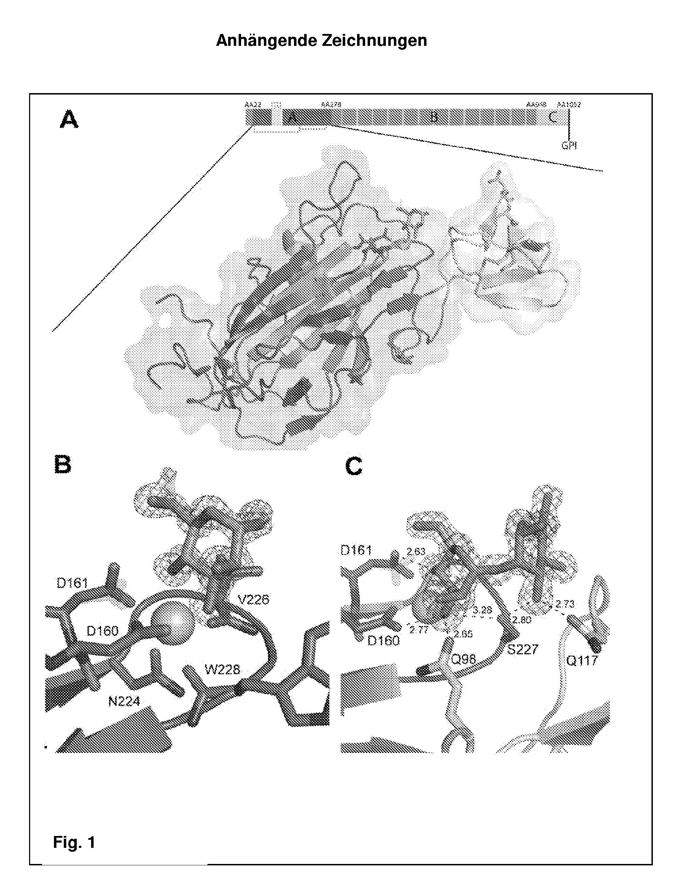

Fig. 1 zeigt in Teil A die Architektur des Flo 5 Gen mit der N-terminalen A-Domäne, die B-Region mit repetitiven hoch glykosylierten repeats und die C-Region, die den Kontakt zur Zellwand über einen GPI-Anker vermittelt.

Teil B zeigt einen möglichen Liganden der Flo5A-Domäne mit Aminosäuren, die direkt in die Calcium- und/oder Substraterkennung involviert sind und die daher erfindungsgemäß mutiert wurden, insbesondere Flo5A-N224, Flo5A-D160, Flo5A-D161, Flo5A-W228, Flo5A-V226.

Teil C zeigt einen möglichen Liganden der Flo5A-Domäne mit Aminosäuren, die direkt in die Calcium- und/oder Substraterkennung involviert sind und die daher erfindungsgemäß mutiert wurden, insbesondere Flo5A-Q98, Flo5A-D160, Flo5A-D161, Flo5A-S227, Flo5A-Q117.

Fig. 2 zeigt den prozentualen Anteil der Flokkulation im Wildtyp-Protein mit der N-terminalen A-Domäne und mit Proteinen, die die Mutationen Flo5A-Q98A, Flo5A-D202T, Flo5A-N224A tragen. Gemessen wurde jeweils in Abwesenheit von Zucker, in Gegenwart von 0.8 m Mannose bzw. in Gegenwart von 0.8 m Glucose.

Fig. 3 zeigt die Kristallografie-Analyse der Flo5A-Domäne mit der Position der Aminosäure D202.

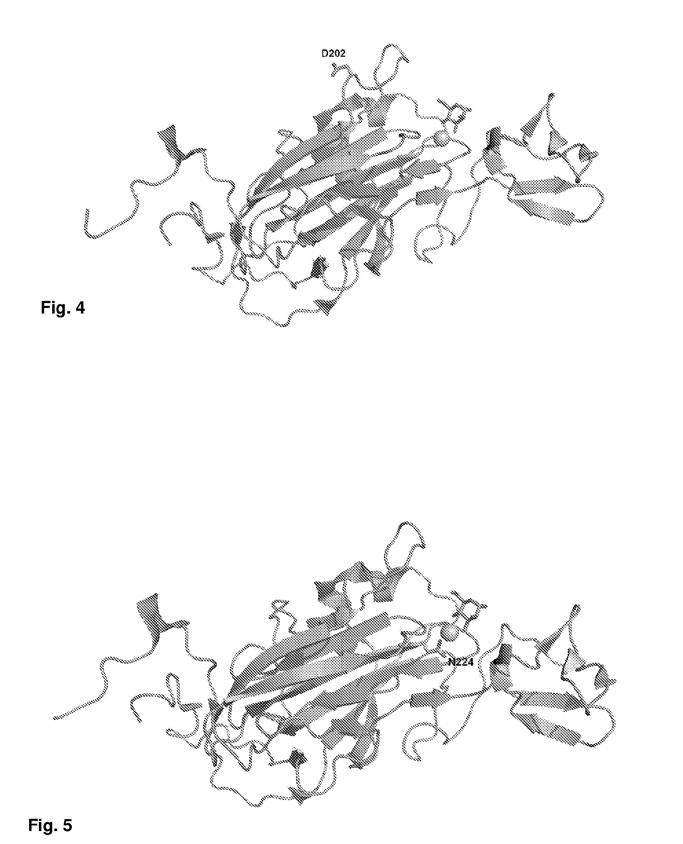

Fig. 4 zeigt die Kristallografie-Analyse der Flo5A-Domäne mit der Position der Aminosäure N224.

Fig. 5 zeigt die Kristallografie-Analyse der Flo5A-Domäne mit der Position der Aminosäure S227.

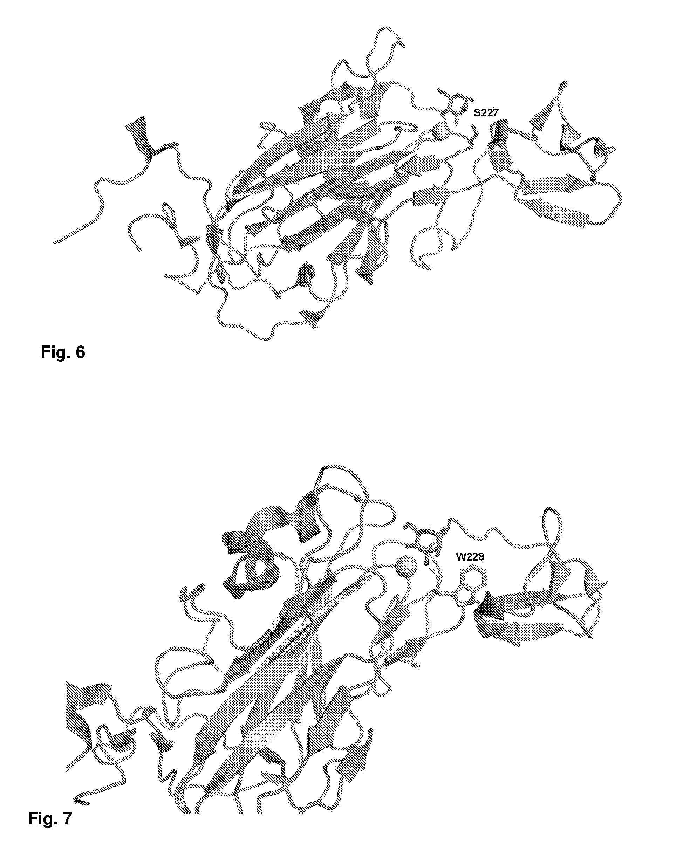

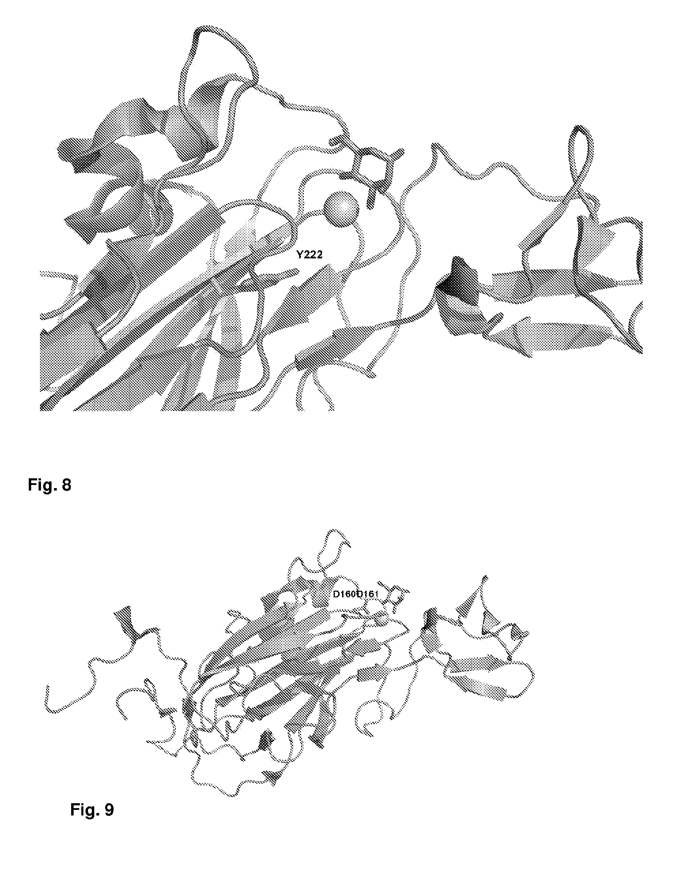

Fig. 6 zeigt die Kristallografie-Analyse der Flo5A-Domäne mit der Position der Aminosäure W228.

Fig. 7 zeigt die Kristallografie-Analyse der Flo5A-Domäne mit der Position der Aminosäure Y222.

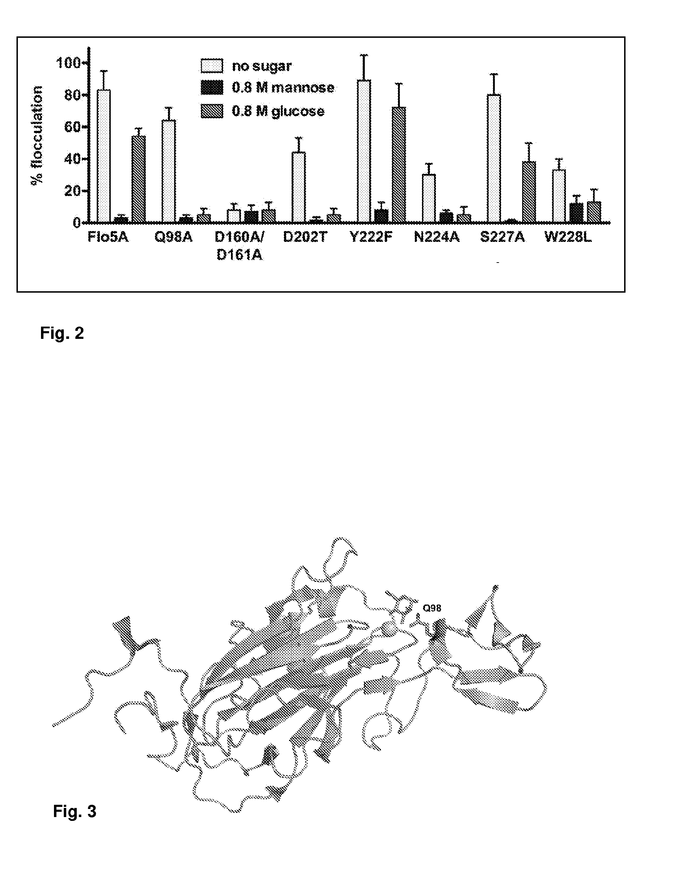

Fig. 3 zeigt die Kristallografie-Analyse der Flo5A-Domäne mit der Position der Aminosäuren D160 und D161.

## Patentansprüche

1. Flokkulationsprotein Flo5 **dadurch gekennzeichnet dass** es eine N-terminale A-Domäne gemäß Sequenz ID No:1 aufweist.

2. Flokkulationsprotein Flo5 **dadurch gekennzeichnet dass** es eine Aminosäuremutation in der N-terminalen A-Domäne aufweist und eine Aminosäuresequenz ausgewählt aus einer der Sequenz ID No: 2 bis Sequenz ID No: 8

3. Flokkulationsprotein Flo5 gemäß Anspruch 2, **dadurch gekennzeichnet dass** die Aminosäuremutation in der N-terminalen A-Domäne Q98A gemäß Sequenz ID No:2 zu einer Flokkulation führt, die durch Mannose und Glucose inhibiert werden kann.

4. Hefezelle aufweisend ein Flokkulationsprotein Flo5 gemäß Anspruch 1.

5. Hefezelle aufweisend ein Flokkulationsprotein Flo5 gemäß Anspruch 2.

6. Hefezelle gemäß Anspruch 4 und 5 **dadurch gekennzeichnet dass** sie ausgewählt ist aus der Gruppe Saccharomyces cerevisiae, Saccharomyces carlsbergensis, Saccharomyces uvarum, Schizosaccharomyces pombe, Candida utilis und Pichia pastoris.

7. Verfahren zur Detektion von Flo5A-tragenden Zellen gemäß Anspruch 4 bis 6 **dadurch gekennzeichnet dass** Hefezellen auf einer Mikrotiterplatte immobilisiert sind und mittels für die Flo5A-Domäne spezifischem Antikörper, der über einen markierten Zweitantikörper, der spezifisch Epitope des für die Flo5A-Domäne spezifischen Antikörpers erkennt und bindet nachgewiesen wird

8. Verfahren gemäß Anspruch 7 **dadurch gekennzeichnet dass** der Zweitantikörper eine fluoreszierende Markierung trägt.

9. Verfahren gemäß Anspruch 7 **dadurch gekennzeichnet dass** der Zweitantikörper eine Markierung in Form von fluoreszierender Markierung trägt
